# EUROPÄISCHE PATENTSCHRIFT

(11) **EP 1 784 128 B1**
(45) Veröffentlichungstag und Bekanntmachung des Hinweises auf die Patenterteilung: **25.06.2014**
(21) Anmeldenummer: 05777650.2
(22) Anmeldetag: 23.08.2005
(51) Int. Cl.: A61B 5/151

(54) **LANZETTENVORRICHTUNG ZUM ERZEUGEN EINER EINSTICHWUNDE**
LANCET DEVICE FOR MAKING A PUNCTURE WOUND
DISPOSITIF A LANCETTES POUR PRODUIRE UNE INCISION

(30) Priorität: 04.09.2004 DE 102004042886
(43) Veröffentlichungstag der Anmeldung: 16.05.2007
(73) Patentinhaber: F.Hoffmann-La Roche AG, 4070 Basel (CH); Roche Diagnostics GmbH, 68305 Mannheim (DE)
(72) Erfinder: WEISS, Thomas, 68307 Mannheim (DE); FORSTER, Richard, 92269 Fensterbach (DE); SACHSENWEGER, Peter, 93197 Zeitlarn (DE)
(74) Vertreter: Petirsch, Markus
(86) Internationale Anmeldenummer: PCT/EP2005/009089
(87) Internationale Veröffentlichungsnummer: WO 2006/027101

(56) Entgegenhaltungen:
- EP-A- 1 384 438
- WO-A-01/00090
- WO-A-02/36010
- DE-A1- 10 047 419
- US-A1- 2003 199 911
- US-B1- 6 228 100

## Beschreibung

Die Erfindung betrifft eine Lanzettenvorrichtung zum Erzeugen einer Einstichwunde, insbesondere zur Gewinnung einer Blutprobe, die für medizinische Zwecke analysiert werden soll. Sie umfaßt eine Magazinaufnahme für ein Lanzettenmagazin mit mehreren Lanzetten, einen Lanzettenantrieb mit einer Antriebsfeder, einer Spanneinrichtung zum Spannen der Antriebsfeder und einer Schubstange, die an eine in dem Lanzettenmagazin angeordnete Lanzette ankuppelbar ist und die mittels des Lanzettenantriebs mit einer daran angekuppelten Lanzette bei einer Einstich- und Rückführbewegung mit hoher Geschwindigkeit bewegt werden kann, um eine Einstichwunde zu erzeugen, sowie eine Magazinfortschaltung, mit der das Lanzettenmagazin derartig fortschaltbar ist, daß die in ihm gelagerten Lanzetten nacheinander mit der Schubstange kuppelbar sind.

Die Erfindung betrifft ferner ein Blutentnahmesystem umfassend eine solche Lanzettenvorrichtung und ein dazu passendes Lanzettenmagazin mit mehreren Lanzetten.

Um für analytisch-diagnostische Zwecke eine geringe Menge Blut aus einem Körperteil (meist aus einem Finger oder Ohrläppchen) zu entnehmen, werden Lanzetten verwendet, die zur Erzeugung einer Einstichwunde in das entsprechende Körperteil gestochen werden. Bereits seit langem sind Blutentnahmesysteme verfügbar, die aus einer Lanzettenvorrichtung und zugehörigen, für die jeweilige Lanzettenvorrichtung speziell angepaßten Lanzetten bestehen. In einem Gehäuse der Lanzettenvorrichtung befindet sich ein Lanzettenantrieb, durch den eine Lanzette mechanisch in die Haut gestochen wird.

An Lanzettenvorrichtungen werden schwierige und teilweise gegenläufige Anforderungen nach minimalem Schmerzempfinden, einfacher Bedienbarkeit, kompakter Bauweise und kostengünstiger Konstruktion gestellt. Um diese Anforderungen möglichst weitgehend zu erfüllen, wurden umfangreiche Entwicklungsarbeiten unternommen. Eine Richtung der Entwicklungsanstrengungen geht dahin, dem Anwender mehr Komfort zu bieten. Eine einfache und komfortable Bedienung ist insbesondere für Diabetiker wichtig, die ihren Blutzuckerspiegel häufig kontrollieren sollten, um ihn durch Insulininjektionen innerhalb bestimmter Sollgrenzen halten zu können. Da die regelmäßige Selbstkontrolle ("Home-Monitoring") des Blutzuckers das Risiko schwerwiegender Langzeitschäden des diabetes mellitus (z. B. Retinopathien, die zur Erblindung führen) wesentlich reduziert und jede Verbesserung des Bedienungskomforts die Bereitschaft der Patienten zur regelmäßigen Selbstkontrolle erhöht, ist der Anwendungskomfort der Systeme auch von sehr großer medizinischer Bedeutung.

Eine Möglichkeit, den Komfort zu erhöhen und die Bedienung einer Lanzettenvorrichtung zu erleichtern, besteht in der Verwendung von Lanzettenmagazinen, die mehrere Lanzetten enthalten und in eine Magazinaufnahme einer Lanzettenvorrichtung eingebracht werden. Auf diese Weise entfällt das Einlegen einzelner Lanzetten, das insbesondere für kranke oder gebrechliche Personen, deren manuelle Geschicklichkeit eingeschränkt ist, mühsam ist.

In der DE 10022720 A1 ist ein Blutentnahmesystem mit einem trommelförmigen Revolvermagazin beschrieben, das ringförmig angeordnet zehn Lanzetten enthält. Dabei erfolgt die Magazinfortschaltung, d.h. der Weitertransport des Magazins der Lanzettenvorrichtung in die nächste Stellung mit einer frischen Lanzette, automatisch nach jedem Einstich. Ein Revolvermagazin mit einer Mehrzahl ringförmig angeordneter Lanzetten wird auch bei dem in der EP 1384438 A1 beschriebenen Blutentnahmesystem verwendet, wobei über dessen Fortschaltung keine Angaben gemacht werden.

Auch in der WO 01/00090 A1 wird ein mit einem Lanzettenmagazin ausgestattetes Blutentnahmesystem beschrieben. Es weist einen Mechanismus zum automatischen schrittweisen Fortschalten des Magazins auf, wobei es als vorteilhaft angesehen wird, daß dadurch zwangsläufig bei jedem neuen Einstich eine frische Lanzette verwendet werden muß. Ähnliches gilt auch für das US-Patent 6,228,100, gemäß dem mit unterschiedlichen Konfigurationen erreicht werden kann, daß sequentiell nach jedem Auslösen des Lanzettenantriebs eine unbenutzte Lanzette in die Benutzungsposition gebracht und dadurch die Wiederverwendung benutzter Lanzetten verhindert wird.

Auch bei dem in der WO 03/071940 A1 und dem in dem US-Patent 6,530,892 beschriebenen Lanzettensystem wird Wert darauf gelegt, daß jeweils zwischen zwei Einstichvorgängen eine Fortschaltung des Lanzettenmagazins derartig erfolgt, daß jeweils eine frische Lanzette zur Verfügung steht.

Trotz dieser vielfältigen Entwicklungsbemühungen konnten sich Lanzettenvorrichtungen mit Lanzettenmagazinen bisher am Markt nicht etablieren. Aufgabe der Erfindung ist es deshalb, eine Magazin-Lanzettenvorrichtung zu schaffen, die von den Anwendern besser akzeptiert wird.

Diese Aufgabe wird durch eine Lanzettenvorrichtung nach Anspruch 1 gelöst.

Die Entwickler der oben diskutierten aus dem Stand der Technik bekannten Blutlanzettensysteme sind übereinstimmend davon ausgegangen, daß es für die Konstruktion und Verwendung von Magazin-Blutlanzettensystemen vorteilhaft sei, wenn jeweils zwischen zwei Einstichen eine Fortschaltung des Magazins erfolgt und demzufolge jeweils eine neue Blutlanzette zur Verfügung steht. Deswegen wurde die Fortschaltung des Magazins mit dem Lanzettenantrieb derartig gekoppelt, daß die Magazinfortschaltungen zwangsweise alternierend mit den Einstichbewegungen stattfinden. In der Regel ist die Kopplung mechanisch. Bei dem US-Patent 6,530,892 ist die Kopplung im Rahmen einer elektronischen Steuerung der Systemfunktionen realisiert.

Im Rahmen der vorliegenden Erfindung wurde gefunden, daß die relativ aufwendige Kopplungskonstruktion für die praktische Handhabung weder notwendig noch besonders vorteilhaft ist. Vielmehr wird im Bereich des Home-Monitoring die Handhabung vereinfacht und gleichzeitig eine vereinfachte Konstruktion erreicht, wenn die Funktionen des Lanzettenantriebs und der Magazinfortschaltung getrennt werden. Es ist auf einfache Weise möglich, die Funktionen "Einstellen der Stechtiefe" und "Fortschalten des Lanzettenmagazins" räumlich zu trennen und so zu lokalisieren, daß eine intuitive Bedienung möglich ist: Stechtiefeneinstellung am Vorderende des Gerätes in der Nähe der Austrittsöffnung der Lanzette; Magazinfortschaltung am hinteren Ende, insbesondere kombiniert mit dem gleichen Betätigungselement, mit dem der Lanzettenantrieb gespannt wird.

Die genannten und weitere Vorteile werden insbesondere erreicht, wenn zusätzlich die nachfolgend anhand eines Ausführungsbeispiels erläuterten bevorzugten Ausgestaltungen zumindest teilweise berücksichtigt werden. Eine erhebliche zusätzliche Kosteneinsparung wird dadurch erreicht, daß der Benutzer selbst bestimmen kann, wie häufig er das Magazin fortschaltet und damit eine neue Lanzette zum Einsatz bringt.

Weitere Einzelheiten und Vorteile der Erfindung werden im folgenden anhand eines Ausführungsbeispiels unter Bezugnahme auf die beigefügten Zeichnungen erläutert. Die dargestellten Besonderheiten können einzeln oder in Kombination verwendet werden, um bevorzugte Ausgestaltungen der Erfindung zu schaffen. Es zeigen:
- Fig. 1: eine erfindungsgemäße Lanzettenvorrichtung in einer Seitenansicht,
- Fig. 2: die Lanzettenvorrichtung in einer Explosionsdarstellung,
- Fig. 3: Teile der Lanzettenvorrichtung in einem aufgeschnittenen Gehäuse und
- Fig. 4: eine Darstellung ähnlich Figur 3, jedoch mit nicht-geschnittener Abbildung einiger Einbauteile der Lanzettenvorrichtung.

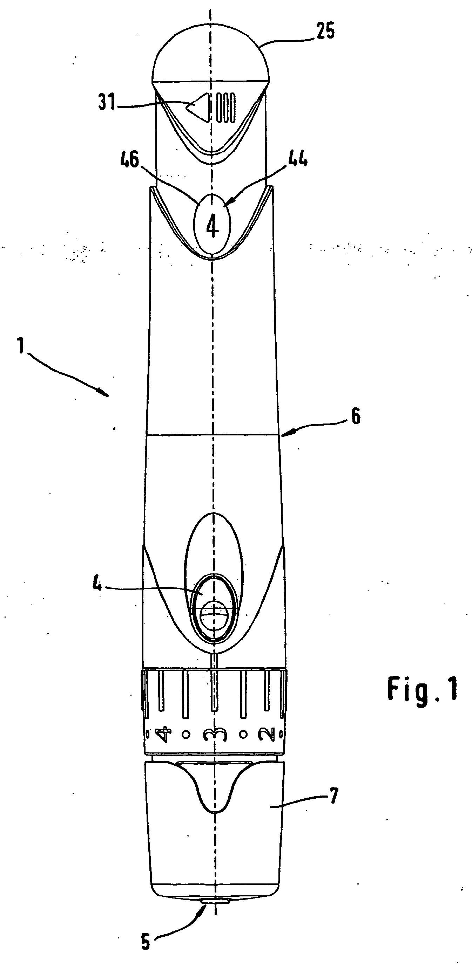

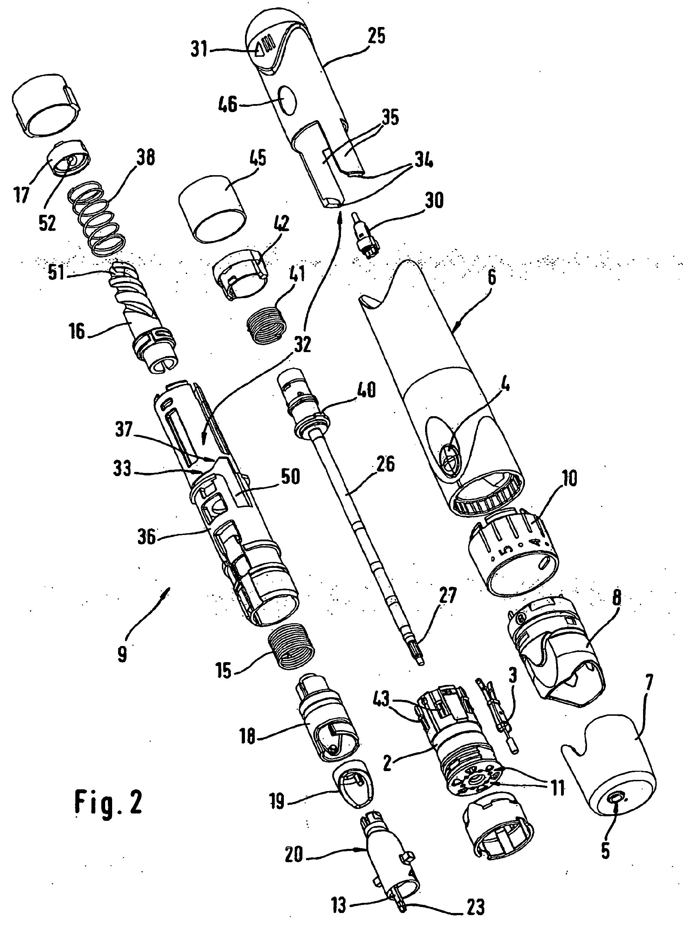

Das in den Figuren 1 und 2 gezeigte Blutentnahmesystem umfaßt eine Lanzettenvorrichtung 1 und ein dazu passendes Lanzettenmagazin 2 mit mehreren Lanzetten 3. Es dient zum Erzeugen einer Einstichwunde zum Entnehmen von Blut für Diagnosezwecke. Durch Druck auf ein Auslöseelement 4 wird die Bewegung eines Lanzettenantriebs 9 der Lanzettenvorrichtung 1 ausgelöst, so daß die Spitze einer Lanzette 3 mit hoher Geschwindigkeit aus einer Austrittsöffnung 5 des Gehäuses 6 austritt. Bei dem gezeigten Ausführungsbeispiel ist die Austrittsöffnung 5 in einer zu dem Gehäuse 6 gehörenden Kappe 7 angeordnet, die ein in eine Magazinaufnahme 8 eingelegtes Lanzettenmagazin 2 umgibt. Ein Stechtiefenverstellring 10 dient zum Einstellen einer hinsichtlich des Schmerzempfindens und der bei einem Einstich gewonnenen Blutmenge optimalen Stechtiefe.

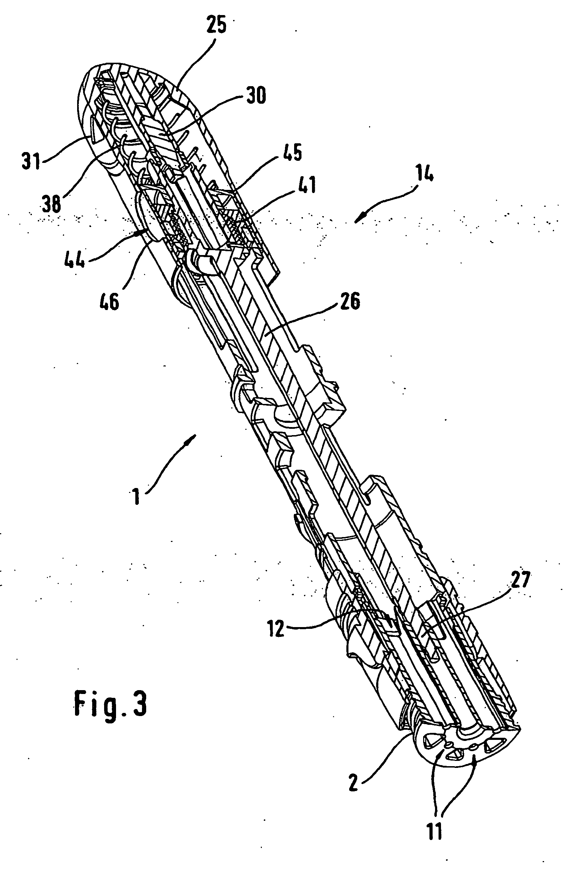

Wie Figur 2 zeigt, ist das Lanzettenmagazin 2 als (trommelförmiges) Revolvermagazin ausgebildet und enthält mehrere, bevorzugt sechs, Lanzetten 3. Die Lanzetten 3 befinden sich in ringförmig angeordneten Lanzettenaufnahmen des Lanzettenmagazins 2. Zu jeder Lanzettenaufnahme gehört eine Lanzettenöffnung 11, die durch Drehung des Lanzettenmagazins 2 fluchtend mit der Austrittsöffnung 5 in einer Kupplungsposition positionierbar ist. Jeder Lanzettenöffnung 11 liegt eine Einschuböffnung 12 (siehe Figur 3) für eine Schubstange 13 des Lanzettenantriebs gegenüber, die in der Kupplungsposition an eine der Lanzetten 3 ankuppelbar ist.

Der Lanzettenantrieb 9 umfaßt eine Antriebsfeder 15, eine Spanneinrichtung 16,17 zum Spannen der Antriebsfeder 15 sowie die Schubstange 13, die mittels des Lanzettenantriebs 9 zusammen mit einer daran angekuppelten Lanzette 3 mit hoher Geschwindigkeit derartig bewegt werden kann, daß eine Einstich- und Rückführbewegung ausgeführt wird.

Wie man in Figur 2 sieht, sind weitere Elemente des Lanzettenantriebs 9 ein durch die Antriebsfeder 15 antreibbarer Antriebsrotor 18 und ein abtriebseitiger Kupplungsmechanismus 19,20, durch den in einer Vortriebsphase des Lanzettenantriebs eine Drehbewegung des Antriebsrotors 18 in eine Translationsbewegung der Schubstange 13 und über diese in eine Einstichbewegung der Lanzette 3 umgesetzt wird.

Die Spanneinrichtung schließt einen drehbeweglichen Spannrotor 16 ein, an dem sich das von dem Antriebsrotor 18 abgewandte Ende der Antriebsfeder 15 abstützt.

Der Spannrotor 16 ist zum Spannen der Antriebsfeder 15 bei gehemmter Rotation des Antriebsrotors 18 in die gleiche Drehrichtung drehbar, in die sich der Antriebsrotor 18 während der Vortriebsphase dreht. Der Spannrotor 16 ist während der Vortriebsphase gegen eine Rückwärtsdrehung arretiert, so daß der Antriebsrotor 18 nach Freigabe der Hemmung eine Drehbewegung durchführt, die in eine Translationsbewegung der Schubstange 13 umgesetzt wird. Der Antriebsrotor durchläuft bei einem Arbeitszyklus, also bei einem Gebrauch des Gerätes, einen Gesamtdrehwinkelbereich von 360°.

Die Spanneinrichtung wird über ein Drehschiebegetriebe 51,52 betätigt, das mit einem Betätigungselement 25 derartig zusammenwirkt, daß die Antriebsfeder 15 beim Niederdrücken des Betätigungselementes 25 gespannt wird. Wie Figur 2 zeigt, wird das Drehschiebegetriebe von einer Steuerkurve 51 in Form einer umlaufenden Doppelnut auf dem Spannrotor 16 und zwei Steuerzapfen 52 der Spannhülse 17 gebildet, von denen die Steuerkurve 51 beim Niederdrücken des Betätigungselements 25 abgefahren wird.

Ein derartiger Lanzettenantrieb ist in der EP 1384438 A1 beschrieben. Dort wird die Bezeichnung OWADAC (One Way Alternating Drive And Cocking) verwendet. Der Inhalt dieses Dokuments wird hinsichtlich weiterer Merkmale und Funktionen des Lanzettenantriebs durch Bezugnahme zum Gegenstand der vorliegenden Anmeldung gemacht.

Wie bereits erwähnt, weist das Lanzettenmagazin 2 gegenüberliegend zu jeder Lanzettenöffnung 11 eine Einschuböffnung 12 auf, durch die die Schubstange 13 zum Ankuppeln an eine Lanzette 3 in das Lanzettenmagazin 2 hineingeschoben werden kann. Die entsprechende Position der Lanzettenaufnahme mit einer Lanzette 3 wird als Kupplungsposition bezeichnet. Die Schubstange 13 wird mit ihrem verdickten Kopfteil 23 formschlüssig an die Lanzette 3 angekuppelt, so daß sie während der Einstich- und Rückführbewegung mit der Schubstange 13 verbunden bleibt. Nähere Einzelheiten und alternative Ausführungsformen eines geeigneten Kupplungsmechanismus sind in der WO 02/36010 A1 beschrieben, die diesbezüglich durch Bezugnahme zum Gegenstand der vorliegenden Anmeldung gemacht wird.

Das Drehen des Lanzettenmagazins 2 um einen Rotationsschritt, bei dem jeweils die nächste Lanzette 3 in die Kupplungsposition gebracht wird, wird als Fortschalten oder Weitertakten bezeichnet und mit einer Magazinfortschaltungs-Mechanik erreicht, die im folgenden näher beschrieben wird.

Die Magazinfortschaltung 14 ist mechanisch von dem Lanzettenantrieb entkoppelt, wobei die Lanzettenvorrichtung 1 ein Betätigungselement 25 aufweist, mit dem die Magazinfortschaltung unabhängig von dem Lanzettenantrieb, insbesondere unabhängig von der Spanneinrichtung 16,17 betätigbar ist. Auf diese Weise kann der Benutzer selbst bestimmen, wie häufig er das Magazin fortschaltet und dadurch eine neue Lanzette zum Einsatz bringt.

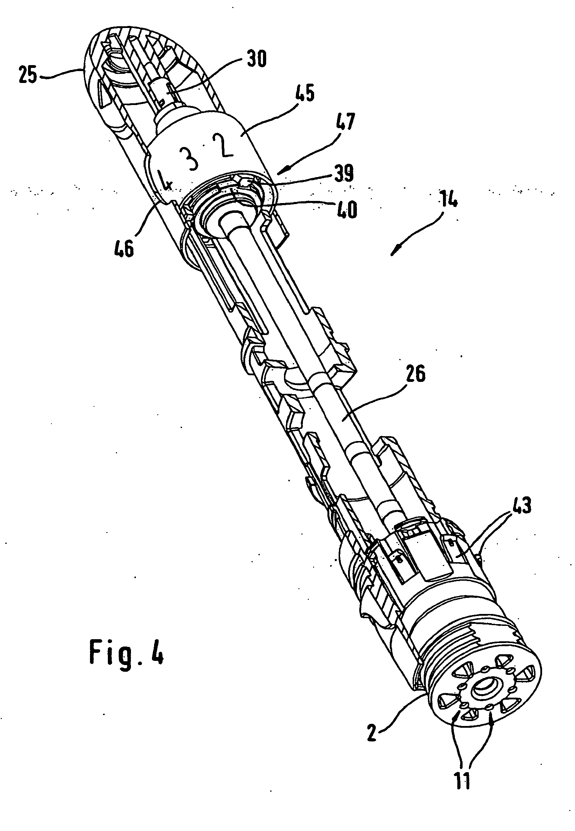

Die Elemente der Magazinfortschaltungs-Mechanik sind im zusammengebauten Zustand in den Figuren 3 und 4 gezeigt, in denen der besseren Übersichtlichkeit halber der Lanzettenantrieb nicht dargestellt ist. Zu der Magazinfortschaltung 14 gehört eine Achse 26, auf die das Lanzettenmagazin 2 aufgesteckt ist und mittels der es gedreht wird. Die Achse 26 weist einen Kopf 27 mit einem Zahnkranz auf, um eine drehfeste Kopplung mit dem Lanzettenmagazin 2 zu gewährleisten. Die Magazinfortschaltung wird durch eine Drehbewegung des Betätigungselements 25 betätigt. Diese Drehbewegung wird über ein Kupplungsteil 30 auf die Achse 26 übertragen. Alternativ kann die Magazinfortschaltung auch durch eine translatorische Bewegung des Betätigungselements, die beispielsweise durch ein Drehschiebegetriebe in eine Drehbewegung der Achse 26 umgewandelt wird, betätigt werden.

Die Magazinfortschaltung 14 ist so ausgebildet, daß das Lanzettenmagazin 2 nur in eine Richtung fortschaltbar ist. Zu diesem Zweck schließt sie eine richtungsgeschaltete Kupplung mittels des Kupplungsteils 30 ein, das zwischen der Achse 26 und dem Betätigungselement 25 angeordnet ist. Bevorzugt ist die richtungsgeschaltete Kupplung 30 als Klinkenfreilauf, insbesondere als Ratsche, ausgebildet. Über die richtungsgeschaltete Kupplung 30 kann eine Drehbewegung des Betätigungselements 25 nur in der einen Richtung auf die Achse 26 übertragen werden. Um einem Benutzer die richtige Drehrichtung anzuzeigen, ist auf dem Betätigungselement 25 eine pfeilförmige proflierte Markierung 31 angebracht.

Damit bei Betätigung der Magazinfortschaltung das Lanzettenmagazin 2 jeweils nur soweit gedreht wird, daß die jeweils nächste Lanzette 3 in ihre Kupplungsposition gelangt, in der sie mit der Schubstange 13 kuppelbar ist, umfaßt die Magazinfortschaltung Drehschrittbegrenzungsmittel 32, die im folgenden anhand von Figur 2 erläutert werden.

Bei dem gezeigten Ausführungsbeispiel sind die Drehschrittbegrenzungsmittel 32 durch eine Kurvensteuerung mit einer Steuerkurve 33 und einem Steuerkurvenreiter 34 realisiert. Die Steuerkurve 33 ist als gegenüber dem Gehäuse drehfeste Nut ausgebildet, die sich in Umfangsrichtung nur über einen hinreichend kleinen Winkelbereich, d.h. weniger als das Doppelte eines Rotationsschritts der Magazinfortschaltung 14, erstreckt. Zur Vermeidung von Kippmomenten sind gegenüberliegend zwei derartige Nuten 33 und zwei Steuerkurvenreiter 34 vorhanden. Da das Lanzettenmagazin des gezeigten Ausführungsbeispiels sechs Lanzettenaufnahmen aufweist, beträgt ein Rotationsschritt 60°. Die Nut 33 erstreckt sich deshalb nur über einen Umfangsbereich von bevorzugt 70 bis 90°, also deutlich weniger als 120°.

In jede Nut 33 greift einer der Steuerkurvenreiter 34 ein, der sich jeweils am Ende von zwei Führungsarmen 35 des Betätigungselements 25 befindet. Bei einer Drehbewegung des Betätigungselements 25 werden die Steuerkurvenreiter 34 von der jeweiligen Nut 33 geführt, bis sie am Ende der Nut 33 anschlagen, so daß eine Drehung des Betätigungselements 25 über das zulässige Maß hinaus ausgeschlossen ist. Die Steuerkurve 33 kann im einfachsten Fall in dem Gehäuse 6 selbst positioniert sein. Bei dem gezeigten Ausführungsbeispiel ist sie in einer drehfest mit dem Gehäuse verbundenen Hülse 36 ausgebildet.

Wie man in Figur 2 erkennt, läuft die Steuerkurve 33 nicht auf voller Länge exakt in Umfangsrichtung, sondern in einem Anfangsbereich leicht schräg. Durch die Schrägung 37 wird das Betätigungselement 25 zu Beginn einer Drehbewegung leicht in axialer Richtung bewegt und dabei gegen die Achse 26 gedrückt. Dadurch wird die Achse 26 über das Kupplungsteil 30 mit dem Betätigungselement 25 in Eingriff gebracht, so daß eine Drehbewegung des Betätigungselements 25 auf die Achse 26 übertragen werden kann.

Vor einer erneuten Betätigung der Magazinfortschaltung wird das Betätigungselement 25 in seine ursprüngliche Position zurückgesetzt. Dies kann dadurch geschehen, daß der Benutzer das Betätigungselement 25 von Hand zurückdreht. Um die Handhabung der Lanzettenvorrichtung 1 so einfach wie möglich zu gestalten, geschieht dies bei dem gezeigten Ausführungsbeispiel automatisch mittels einer Feder 38. Die Feder 38 kann eine Torsionsfeder sein, die einer Drehbewegung des Betätigungselements entgegenwirkt. Bevorzugt wird eine Kompressionsfeder verwendet, die bei der mit einer Drehung verbundenen Federbewegung in axialer Richtung komprimiert wird. Wegen der Schräge 37 der Steuerkurve 33 wird einem Benutzer das Zurücksetzen des Betätigungselements 25 durch die in axialer Richtung wirkende Federkraft der Feder 38 erleichtert.

Die Magazinfortschaltung 14 schließt eine in Figur 4 erkennbare Umlaufsperre 40 ein, durch welche die Rotation des Lanzettenmagazins auf weniger als 360° beschränkt ist. Diese Maßnahme hat den Vorteil, daß nicht versehentlich eine bereits vor geraumer Zeit benutzte Lanzette 3 für eine frische Lanzette 3 gehalten und erneut verwendet wird. Zwischen der Benutzung der ersten Lanzette 3 eines Lanzettenmagazins 2 und der letzten können unter Umständen mehrere Tage vergehen. In einem so langen Zeitraum können sich auf einer benutzten Lanzette 3 aus der Umgebung stammende Krankheitserreger und Sporen in so starkem Maß vermehren, daß selbst bei Benutzung durch dieselbe Person eine Gesundheitsgefahr besteht. Bei der von vielen Diabetikern im Rahmen ihrer regelmäßigen Selbstkontrolle des Blutzuckergehalts praktizierten zwei- oder dreifachen Verwendung einer Lanzette in einem Zeitraum von wenigen Stunden besteht demgegenüber praktisch keine Infektionsgefahr.

Bei dem gezeigten Ausführungsbeispiel ist die Umlaufsperre 47 mittels eines an der Achse angeordneten Anschlagelementes 40 realisiert, das mit einem gegenüber dem Gehäuse 6 drehfesten Anschlag 39 zusammenwirkt. Der Anschlag 39 könnte im einfachsten Fall an dem Gehäuse 6 selbst positioniert sein, ist bei dem gezeigten Ausführungsbeispiel jedoch Teil einer die Achse 26 umgebenden Hülse.

Durch eine in den Figuren 2 und 3 gezeigten Rückstellfeder 41, die bevorzugt unter Vorspannung steht, wird erreicht, daß sich die Achse 26 bei jedem Wechsel des Lanzettenmagazins 2 in ihre ursprüngliche Position zurückdreht und folglich die Umlaufsperre 47 erst anspricht, wenn die Achse 26 und damit auch das Lanzettenmagazin 2 so weit gedreht wurde, daß alle Lanzetten 3 benutzt werden konnten. Bei der Rückstellfeder handelt es sich um eine Torsionsfeder 41, die um die Achse 26 herum angeordnet ist. Sie stützt sich mit einem Ende an der Achse 26 und mit dem anderen Ende an einer drehfest mit dem Gehäuse 6 verbundenen Stützhülse 42 ab.

Bei Betätigung der Magazinfortschaltung, also bei jedem Rotationsschritt des Lanzettenmagazins 2, wird die Rückstellfeder 41 zunehmend gespannt. Eine Rücklaufsperre 43 verhindert dabei ein Zurückdrehen des Lanzettenmagazins 2 hinter eine einmal erreichte Kupplungsposition. Bei dem gezeigten Ausführungsbeispiel ist die Rücklaufsperre 43 durch Federbeine an der Mantelfläche des Lanzettenmagazins 2 verwirklicht, die in abgeschrägte Ausnehmungen (nicht dargestellt) der Magazinaufnahme 8 eingreifen. Die Abschrägungen der Ausnehmungen sind dabei so ausgerichtet, daß die Federbeine bei einer Drehung des Lanzettenmagazins 2 in der richtigen Drehrichtung auf der Schrägfläche entlang und aus den Ausnehmungen herausrutschen können, während eine Drehung in entgegengesetzte Richtung durch eine steile Kante oder einen Hinterschnitt verhindert wird, gegen die die Federbeine anschlagen. Bei Entnahme eines Lanzettenmagazins aus der Magazinaufnahme wird die Rücklaufsperre 43 gelöst, so daß die Achse 26 von der Rückstellfeder 41 automatisch in ihre Ausgangsposition zurückgedreht wird.

Wie man insbesondere in den Figuren 1 und 2 erkennt, weist die Lanzettenvorrichtung 1 eine Anzeigeeinrichtung 44 zum Anzeigen der Anzahl der unbenutzten Lanzetten eines in der Magazinaufnahme positionierten Lanzettenmagazins auf. Die Anzeigeeinrichtung umfaßt einen Markierungsring 45, der auf einer einem Gehäusefenster 46 zugewandten Außenseite Markierungen, insbesondere Zahlen, aufweist, die jeweils einer Anzahl unbenutzter Lanzetten des Lanzettenmagazins zugeordnet sind, wobei eine Betätigung der Magazinfortschaltung einen Drehwinkelschritt des Markierungsrings 45 gegenüber dem Gehäusefenster 46 bewirkt, so daß in dem Gehäusefenster 46 eine Markierung des Markierungsrings 45 sichtbar wird, der eine um eins verminderte Anzahl unbenutzter Lanzetten 3 zugeordnet ist.

Anstelle von Zahlen können auf dem Markierungsring beispielsweise auch sich in Umfangsrichtung erstreckende Balken von unterschiedlicher Länge aufgezeichnet sein. Anhand der Anzahl der in dem Fenster sichtbaren Balken kann ein Benutzer dann die Anzahl der unbenutzten Lanzetten 3 erkennen.

Anstelle eines Fensters 46, durch das nur ein Teil der Außenseite des Markierungsrings 45 sichtbar ist, kann auch ein Gehäuseaufbau gewählt werden, bei dem die gesamte Außenseite des Markierungsrings 45 sichtbar ist. In diesem Fall kann mit einer Gehäusemarkierung, beispielsweise einem Pfeil, angezeigt werden, welche der sichtbaren Markierungen des Markierungsrings 45 die Anzahl der unbenutzten Lanzetten angibt. Das bei dem gezeigten Ausführungsbeispiel vorhandene Gehäusefenster 46 ist ebenso wie ein Pfeil eine Gehäusemarkierung, mit der angezeigt wird, welche der sichtbaren Markierungen des Markierungsrings 45 die Anzahl der unbenutzten Lanzetten angibt.

Bevorzugt ist der Markierungsring 45 drehfest mit der Achse 26 verbunden. Dadurch überträgt sich ein Rotationsschritt des Lanzettenmagazins 2 stets auch auf den Markierungsring 45 und folglich ist eine zuverlässige Anzeige gegeben.

Die Anzeigeeinrichtung 44 kann auch so ausgebildet sein, daß statt der Zahl der verbleibenden unbenutzten Lanzetten die Zahl der bereits verbrauchten Lanzetten angezeigt wird. Generell ist jede Anzeigeeinrichtung geeignet, die dem Benutzer den Benutzungszustand der Lanzetten eines in der Magazinaufnahme enthaltenen Magazins anzeigt.

Das Betätigungselement 25 hat mehrere Betätigungsfunktionen, die voneinander unabhängig sind. Mittels einer ersten Betätigungsfunktion wird - wie beschrieben - die Magazinfortschaltung und mittels einer zweiten Betätigungsfunktion die Spanneinrichtung 16,17 betätigt. Die Magazinfortschaltung wird bei dem gezeigten Ausführungsbeispiel, wie bereits erwähnt, durch Drehen des Betätigungselements 25, die Spanneinrichtung 16,17 durch Niederdrücken betätigt. Auf diese Weise wird die Anzahl der erforderlichen Betätigungselemente auf ein Mindestmaß reduziert und die Handhabbarkeit verbessert.

Eine in axialer Richtung verlaufende Führung 50 sorgt dafür, daß sich das Betätigungselement 25 beim Niederdrücken nicht verdrehen kann, so daß während des Spannvorgangs eine unbeabsichtigte Betätigung der Magazinfortschaltung ausgeschlossen ist. Die Führung 50 ist bei dem gezeigten Ausführungsbeispiel als in axialer Richtung verlaufende Nuten ausgebildet, in die jeweils ein Führungsarm 35 des Betätigungselements 25 eingreift. Je nach Betätigungsfunktion werden die Steuerkurvenreiter 34 also von der Steuerkurve 33 oder den Führungsnuten 50 geführt.

## Patentansprüche

1. Lanzettenvorrichtung zum Erzeugen einer Einstichwunde, umfassend
eine Magazinaufnahme (8) für ein Lanzettenmagazin (2) mit mehreren Lanzetten (3),
einen Lanzettenantrieb mit einer Antriebsfeder (15), einer Spanneinrichtung (16,17) zum Spannen der Antriebsfeder (15) und mit einer Schubstange (13), die an eine in dem Lanzettenmagazin (2) angeordnete Lanzette (3) ankuppelbar ist und die mittels des Lanzettenantriebs (9) zusammen mit einer daran angekuppelten Lanzette (3) bei einer Einstich- und Rückführbewegung mit hoher Geschwindigkeit bewegt werden kann, um eine Einstichwunde zu erzeugen, und
eine Magazinfortschaltung (14), mit der das Lanzettenmagazin (2) so fortschaltbar ist, dass die in ihm gelagerten Lanzetten (3) nacheinander mit der Schubstange (13) kuppelbar sind,
**dadurch gekennzeichnet, dass**
der Lanzettenantrieb von der Magazinfortschaltung (14) entkoppelt ist, wobei die Lanzettenvorrichtung ein Betätigungselement (25) aufweist, mit dem die Magazinfortschaltung (14) unabhängig von dem Lanzettenantrieb, insbesondere unabhängig von der Spanneinrichtung (16,17), derartig betätigbar ist, dass der Benutzer selbst bestimmen kann, wie häufig er das Magazin fortschaltet und damit eine neue Lanzette zum Einsatz bringt.

2. Lanzettenvorrichtung nach Anspruch 1, **dadurch gekennzeichnet, daß** die Magazinfortschaltung (14) so ausgebildet ist, daß das Lanzettenmagazin (2) nur in eine Richtung fortschaltbar ist.

3. Lanzettenvorrichtung nach einem der vorhergehenden Ansprüche, **dadurch gekennzeichnet, daß** das Betätigungselement (25) drehbar ist und die Magazinfortschaltung (14) durch eine Drehbewegung des Betätigungselementes (25) betätigbar ist.

4. Lanzettenvorrichtung nach einem der vorhergehenden Ansprüche, **dadurch gekennzeichnet, daß** das Lanzettenmagazin (2) als Revolvermagazin ausgebildet ist.

5. Lanzettenvorrichtung nach Anspruch 4, **dadurch gekennzeichnet, daß** die Magazinfortschaltung (14) eine Achse (26) umfaßt, über die das Betätigungselement (25) mit dem Lanzettenmagazin (2) gekoppelt ist.

6. Lanzettenvorrichtung nach einem der vorhergehenden Ansprüche, **dadurch gekennzeichnet, daß** die Magazinfortschaltung (14) eine richtungsgeschaltete Kupplung (30) umfaßt, die zwischen der Achse (26) und dem Betätigungselement (25) angeordnet ist.

7. Lanzettenvorrichtung nach Anspruch 6, **dadurch gekennzeichnet, daß** die richtungsgeschaltete Kupplung (30) als Klinkenfreilauf, insbesondere als Ratsche, ausgebildet ist.

8. Lanzettenvorrichtung nach einem der Ansprüche 4 bis 7, **dadurch gekennzeichnet, daß** die Magazinfortschaltung (14) eine Rückstellfeder (41) umfaßt, die bei Betätigung der Magazinfortschaltung gespannt wird.

9. Lanzettenvorrichtung nach einem der Ansprüche 4 bis 8, **dadurch gekennzeichnet, daß** die Magazinfortschaltung (14) eine Umlaufsperre (47) umfaßt, durch die die Rotation des Lanzettenmagazins (2) auf weniger als 360° beschränkt ist.

10. Lanzettenvorrichtung nach einem der vorhergehenden Ansprüche, **dadurch gekennzeichnet, daß** sie eine Anzeigeeinrichtung (44) zum Anzeigen des Benutzungszustandes der Lanzetten (3) eines in die Magazinaufnahme (8) eingelegten Lanzettenmagazins (2) aufweist.

11. Lanzettenvorrichtung nach Anspruch 10, **dadurch gekennzeichnet, daß** die Anzeigeeinrichtung (44) einen Markierungsring (45) einschließt, der auf einer Außenseite Markierungen, vorzugsweise Zahlen, aufweist, die jeweils der Position der Lanzetten des Lanzettenmagazins (2) zugeordnet sind, wobei eine Betätigung der Magazinfortschaltung (14) einen Drehwinkelschritt des Markierungsrings (45) gegenüber einer Gehäusemarkierung (46) bewirkt, so daß die Gehäusemarkierung (46) nach dem Drehwinkelschritt eine weitere Markierung des Markierungsrings (45) anzeigt.

12. Lanzettenvorrichtung nach Anspruch 11, **dadurch gekennzeichnet, daß** der Markierungsring (45) Zahlenmarkierungen aufweist und der nach dem Drehwinkelschritt angezeigten Markierung eine um eins verminderte Anzahl unbenutzter Lanzetten (3) zugeordnet ist.

13. Lanzettenvorrichtung nach einem der vorhergehenden Ansprüche, **dadurch gekennzeichnet, daß** das Betätigungselement (25) mehrere Betätigungsfunktionen hat, die voneinander unabhängig sind, wobei mittels einer ersten Betätigungsfunktion die Magazinfortschaltung (14) und mittels einer zweiten Betätigungsfunktion die Spanneinrichtung (16,17) betätigt wird.

14. Lanzettenvorrichtung nach Anspruch 13, **dadurch gekennzeichnet, daß** eine der beiden Betätigungsfunktionen, vorzugsweise die erste Betätigungsfunktion, durch Drehen des Betätigungselementes (25) auslösbar ist und die andere der beiden Betätigungsfunktionen, vorzugsweise die zweite Betätigungsfunktion, durch Niederdrücken des Betätigungselementes (25) auslösbar ist.

15. Lanzettenvorrichtung nach einem der vorhergehenden Ansprüche, **dadurch gekennzeichnet, daß** der Lanzettenantrieb einen durch die Antriebsfeder (15) antreibbaren Antriebsrotor (18) und einen abtriebsseitigen Kuppiungsmechanismus (19,20) umfaßt, durch den in einer Vortriebsphase des Lanzettenantriebs eine Drehbewegung des Antriebsrotors (18) in eine Translationsbewegung der Schubstange (13) und über diese in eine Einstichbewegung der Lanzette (3) umgesetzt wird.

16. Lanzettenvorrichtung nach einem der vorhergehenden Ansprüche, **dadurch gekennzeichnet, daß** die Spanneinrichtung (16,17) ein Drehschiebegetriebe (50,51) umfaßt, das mit dem Betätigungselement (25) derartig zusammenwirkt, daß die Antriebsfeder (15) beim Niederdrücken des Betätigungselementes (25) gespannt wird.

17. Lanzettenvorrichtung nach einem der vorhergehenden Ansprüche, **dadurch gekennzeichnet, dass** die Magazinfortschaltung (14) ein Begrenzungsmittel (32) aufweist, durch welches das Lanzettenmagazin (2) bei Betätigung der Magazinfortschaltung (14) derart begrenzt wird, dass die jeweils nächste Lanzette (3) in ihre Kupplungsposition gelangt, in der sie mit der Schubstange (13) kuppelbar ist.

18. Blutentnahmesystem umfassend eine Lanzettenvorrichtung (1) nach einem der vorhergehenden Ansprüche und ein dazu passendes Lanzettenmagazin (2) mit mehreren Lanzetten (3).

## Claims

1. A lancing apparatus for producing a puncture wound, comprising
a magazine holder (8) for a lancet magazine (2) with a plurality of lancets (3),
a lancet drive comprising a drive spring (15), a tensioning device (16, 17) for tensioning the drive spring (15) and comprising a push rod (13), said push rod (13) being adapted for coupling to a lancet (3) located in the lancet magazine (2) and said push rod (13) being adapted for moving at high velocity, together with a lancet (3) coupled thereto, by means of lancet drive (9) during a puncture and retraction movement producing a puncture wound, and
a magazine advancing mechanism (14) for advancing the lancet magazine (2) in such a manner that the lancets (3) stored therein can be coupled to the push rod (13) one after the other,
**characterized in that**
the lancet drive is decoupled from the magazine advancing mechanism (14), the lancing apparatus comprising an actuating element (25) for actuating the magazine advancing mechanism (14) independently from the lancet drive, in particular independently from the tensioning device (16, 17), in such a manner that the user may determine by himself how often the magazine is advanced and thus a new lancet is applied.

2. The lancing apparatus according to Claim 1, **characterized in that** the magazine advancing mechanism (14) is adapted and arranged for advancing the lancet magazine (2) in one direction only.

3. The lancing apparatus according to any one of the preceding claims, **characterized in that** the actuating element (25) is adapted for rotational movement and the magazine advancing mechanism (14) can be actuated by a rotational movement of the actuating element (25).

4. The lancing apparatus according to any one of the preceding claims, **characterized in that** the lancet magazine (2) is a revolver magazine.

5. The lancing apparatus according to Claim 4, **characterized in that** the magazine advancing mechanism (14) comprises an axle (26), said axle coupling the actuating element (25) to the lancet magazine (2).

6. The lancing apparatus according to any one of the preceding claims, **characterized in that** the magazine advancing mechanism (14) comprises an unidirectional coupling (30), said unidirectional coupling being located between the axle (26) and the actuating element (25).

7. The lancing apparatus according to Claim 6, **characterized in that** the unidirectional coupling (30) comprises a pawl freewheel, preferably a ratchet.

8. The lancing apparatus according to any one of Claims 4 to 7, **characterized in that** the magazine advancing mechanism (14) comprises a restoring spring (41), adapted to be tensioned upon actuation of the magazine advancing mechanism.

9. The lancing apparatus according to any one of Claims 4 to 8, **characterized in that** the magazine advancing mechanism (14) comprises a rotation limitation device (47) for restricting the rotation of the lancet magazine (2) to less than 360°.

10. The lancing apparatus according to any one of the preceding claims, **characterized by** a display (44) for displaying the usage status of the lancets (3) of a lancet magazine (2) inserted into the magazine holder (8).

11. The lancing apparatus according to Claim 10, **characterized in that** the display (44) includes a marking ring (45) having markings, preferably numbers, on an exterior side, each marking being assigned to a position of a lancet in the lancet magazine (2), an actuation of the magazine advancing mechanism (14) causing a rotational angle step of the marking ring (45) in relation to a housing marking (46), so that the housing marking (46) indicates after the rotational angle step a further marking of the marking ring (45).

12. The lancing apparatus according to Claim 11, **characterized in that** the marking ring (45) has numeric markings and the marking indicated after the rotational angle step corresponds to a number of unused lancets (3) which is in each rotational step reduced by one.

13. The lancing apparatus according to any one of the preceding claims, **characterized in that** the actuating element (25) has a plurality of actuating functions, which are independent of one another, the magazine advancing mechanism (14) being actuated using a first actuating function and the tensioning device (16, 17) being actuated using a second actuating function.

14. The lancing apparatus according to Claim 13, **characterized in that** one of the two actuating functions, preferably the first actuating function, can be triggered by rotating the actuating element (25), and the other of the two actuating functions, preferably the second actuating function, can be triggered by pressing down the actuating element (25).

15. The lancing apparatus according to any one of the preceding claims, **characterized in that** the lancet drive comprises a drive rotor (18) adapted to be driven by the drive spring (15) and an output-side coupling mechanism (19, 20), by which, in a forward movement phase of the lancet drive, a rotational movement of the drive rotor (18) is converted into a translational movement of the push rod (13) and thereby into a puncture movement of the lancet (3).

16. The lancing apparatus according to any one of the preceding claims, **characterized in that** the tensioning device (16, 17) comprises a rotary-slide-transmission (50, 51) for cooperation with the actuating element (25) in such a manner that the drive spring (15) is tensioned when the actuating element (25) is pressed down.

17. The lancing apparatus according to any one of the preceding claims, **characterized in that** the magazine advancing mechanism (14) comprises a transport limitation device (32) for limiting the lancet magazine (2) movement upon actuation of the magazine advancing mechanism (14) in such a manner that in each case the lancet (3) which is next in line reaches its coupling position, in which it can be coupled to the push rod (13).

18. A blood sampling system comprising a lancing apparatus (1) according to any one of the preceding claims and a lancet magazine (2) adapted thereto and including a plurality of lancets (3).

## Revendications

1. Dispositif à lancettes pour produire une incision, comprenant
un logement de cartouche (8) pour une cartouche de lancettes (2) contenant plusieurs lancettes (3),
un mécanisme d'entraînement de lancette comprenant un ressort d'entraînement (15), un dispositif tendeur (16, 17) pour tendre le ressort d'entraînement (15) et une tige de poussée (13) qui peut s'accoupler à une lancette (3) placée dans la cartouche de lancettes (2) et qui peut se déplacer au moyen du mécanisme d'entrainement de lancette (9), conjointement avec une lancette (3) qui y est accouplée, à une vitesse élevée lors d'un mouvement d'incision et de retrait, afin de produire une incision, et
un système d'avancement de cartouche (14) qui permet de faire avancer la cartouche de lancettes (2) de manière que les lancettes (3) qui y sont rangées puissent se coupler successivement à la tige de poussée (13),
**caractérisé en ce que**
le mécanisme d'entraînement de lancette est découplé du système d'avancement de cartouche (14), le dispositif à lancettes présentant un élément d'actionnement (25) qui permet d'actionner le système d'avancement de cartouche (14) indépendamment du mécanisme d'entraînement de lancette, en particulier indépendamment du dispositif tendeur (16, 17), de telle sorte que l'utilisateur peut déterminer lui-même à quelle fréquence il fait avancer la cartouche et donc à quelle fréquence il utilise une nouvelle lancette.

2. Dispositif à lancettes selon la revendication 1, **caractérisé en ce que** le système d'avancement de cartouche (14) est conçu de façon que la cartouche de lancettes (2) ne puisse avancer que dans un seul sens.

3. Dispositif à lancettes selon l'une des revendications précédentes, **caractérisé en ce que** l'élément d'actionnement (25) est rotatif et le système d'avancement de cartouche (14) est actionnable par un mouvement de rotation de l'élément d'actionnement (25).

4. Dispositif à lancettes selon l'une des revendications précédentes, **caractérisé en ce que** la cartouche de lancettes (2) est conçue sous forme de magasin rotatif.

5. Dispositif à lancettes selon la revendication 4, **caractérisé en ce que** le système d'avancement de cartouche (14) comprend un axe (26) par l'intermédiaire duquel l'élément d'actionnement (25) est couplé à la cartouche de lancettes (2).

6. Dispositif à lancettes selon l'une des revendications précédentes, **caractérisé en ce que** le système d'avancement de cartouche (14) comprend un accouplement directionnel (30), disposé entre l'axe (26) et l'élément d'actionnement (25).

7. Dispositif à lancettes selon la revendication 6, **caractérisé en ce que** l'accouplement directionnel (30) est conçu sous forme de roue libre à cliquet, en particulier sous forme de dispositif à rochet.

8. Dispositif à lancettes selon l'une des revendications 4 à 7, **caractérisé en ce que** le système d'avancement de cartouche (14) comprend un ressort de rappel (41) qui se tend lorsqu'on actionne le système d'avancement de cartouche.

9. Dispositif à lancettes selon l'une des revendications 4 à 8, **caractérisé en ce que** le système d'avancement de cartouche (14) comprend un dispositif anti-rotation (47) grâce auquel la rotation de la cartouche de lancettes (2) est limitée à moins de 360°.

10. Dispositif à lancettes selon l'une des revendications précédentes, **caractérisé en ce qu'**il présente un moyen de visualisation (44) destiné à indiquer l'état d'utilisation des lancettes (3) d'une cartouche de lancettes (2) placée dans le logement de cartouche (8).

11. Dispositif à lancettes selon la revendication 10, **caractérisé en ce que** le moyen de visualisation (44) comporte une bague de repérage (45) ayant sur un côté extérieur des repères, de préférence des chiffres, associés chacun à la position des lancettes de la cartouche de lancettes (2), un actionnement du système d'avancement de cartouche (14) ayant pour effet un pas de rotation angulaire de la bague de repérage (45) par rapport à un repère sur le boîtier (46), de telle sorte que le repère sur le boîtier (46) indique un autre repère de la bague de repérage (45) après le pas de rotation angulaire.

12. Dispositif à lancettes selon la revendication 11, **caractérisé en ce que** la bague de repérage (45) présentent des repères numériques, et au repère indiqué après le pas de rotation angulaire est associé un nombre moins un de lancettes (3) non utilisées.

13. Dispositif à lancettes selon l'une des revendications précédentes, **caractérisé en ce que** l'élément d'actionnement (25) a plusieurs fonctions d'actionnement qui sont indépendantes les unes des autres, une première fonction d'actionnement permettant d'actionner le système d'avancement de cartouche (14) et une deuxième fonction d'actionnement permettant d'actionner le dispositif tendeur (16, 17).

14. Dispositif à lancettes selon la revendication 13, **caractérisé en ce que** l'une des deux fonctions d'actionnement, de préférence la première fonction d'actionnement, peut être déclenchée en faisant tourner l'élément d'actionnement (25), et l'autre des deux fonctions d'actionnement, de préférence la deuxième fonction d'actionnement, peut être déclenchée en appuyant sur l'élément d'actionnement (25).

15. Dispositif à lancettes selon l'une des revendications précédentes, **caractérisé en ce que** le mécanisme d'entraînement de lancette comprend un rotor d'entraînement (18) actionnable par le ressort d'entraînement (15), et un mécanisme de couplage côté sortie (19, 20) qui, dans une phase d'avancement du mécanisme d'entraînement de lancette, transforme un mouvement de rotation du rotor d'entraînement (18) en un mouvement de translation de la tige de poussée (13) et par l'intermédiaire de celle-ci en un mouvement d'incision de la lancette (3).

16. Dispositif à lancettes selon l'une des revendications précédentes, **caractérisé en ce que** le dispositif tendeur (16, 17) comprend un mécanisme pivotant coulissant (50, 51) qui coopère avec l'élément d'actionnement (25) de manière à tendre le ressort d'entraînement (15) lorsqu'on appuie sur l'élément d'actionnement (25).

17. Dispositif à lancettes selon l'une des revendications précédentes, **caractérisé en ce que** le système d'avancement de cartouche (14) présente un moyen de limitation (32) qui limite la cartouche de lancettes (2) lors de l'actionnement du système d'avance de cartouche (14) de manière que la lancette suivante atteigne sa position de couplage dans laquelle elle peut être couplée à la tige de poussée (13).

18. Système de prélèvement de sang comprenant un dispositif à lancettes (1) selon l'une des revendications précédentes et une cartouche de lancettes adaptée (2) contenant plusieurs lancettes (3).
